Europäisches Patentamt

European Patent Office

Office européen des brevets

(19)

(11) Numéro de publication : **0 389 392 B1**

(12) **FASCICULE DE BREVET EUROPEEN**

(45) Date de publication du fascicule du brevet :
**11.11.92 Bulletin 92/46**

(21) Numéro de dépôt : **90420144.9**

(22) Date de dépôt : **21.03.90**

(51) Int. Cl.$^5$ : **A61F 2/36**

(54) **Prothèse fémorale utilisant une rotule en céramique de petit diamètre.**

(30) Priorité : **23.03.89 FR 8904431**

(43) Date de publication de la demande :
**26.09.90 Bulletin 90/39**

(45) Mention de la délivrance du brevet :
**11.11.92 Bulletin 92/46**

(84) Etats contractants désignés :
**CH DE ES GB IT LI SE**

(56) Documents cités :
**DE-A- 3 023 354**
**FR-A- 1 471 000**
**FR-A- 2 329 249**
**FR-A- 2 610 514**

(56) Documents cités :
**PROC. OF THE INST. OF MECH. ENGI-**
**NEERS/PART H, J. OF ENG. IN MEDICINE, vol.**
**203, no. H1, 1989, pages 1-14, IMechE, B.**
**St.Edmunds, GB; H. FESSLER et al.: "Friction**
**in femoral prosthesis and photoelastic model**
**cone taper joints"**

(73) Titulaire : **CERAMIQUES TECHNIQUES**
**DESMARQUEST**
**Tour Manhattan Place de l'Iris 6 La Défense 2**
**92400 Courbevoie (FR)**

(72) Inventeur : **Prats, Christian**
**5, rue de Dreux**
**F-27000 Evreux (FR)**
Inventeur : **Vivier, Pierre**
**4, rue de Ridder**
**F-75014 Paris (FR)**

(74) Mandataire : **Rinuy, Santarelli**
**14, avenue de la Grande Armée**
**F-75017 Paris (FR)**

## Description

## DOMAINE TECHNIQUE

L'invention concerne une prothèse fémorale constituée d'une tige métallique et d'une tête fémorale ou rotule céramique, de préférence en zircone, assemblées par emmanchement conique. Ladite tête fémorale est en général associée à une cupule acétabulaire en polyéthylène (PE) haute densité.

## ETAT DE LA TECHNIQUE

Lors de l'implantation d'une prothèse totale de hanche, on emploie des têtes fémorales ayant un diamètre généralement compris entre 22,22 et 32 mm selon les techniques chirurgicales mises en oeuvre. Les matériaux utilisés pour la réalisation des têtes fémorales sont de préférence les métaux comme les aciers inox, les alliages Cr-Co-Mo, le titane, etc... ou les céramiques comme l'alumine frittée dense. Une telle céramique présente des avantages démontrés de biocomptabilité et un meilleur coefficient de frottement face au PE haute densité que les métaux. Mais compte tenu de ses propriétés mécaniques, certes élevées, mais insuffisantes, son usage est limité aux têtes fémorales de gros diamètres, supérieurs ou égaux à 28 mm et à des configurations géométriques limitées (essentiellement écart de col maximal de 4 mm pour des têtes fémorales de diamètre 28 mm et de 8 mm pour un diamètre de 32 mm), dans le cas où on veut implanter des prothèses fémorales constituées d'une tige métallique assemblée, par un emmanchement conique, à une rotule d'alumine.

Actuellement si on veut implanter des prothèses ayant des têtes fémorales de petit diamètre fixées par emmanchement conique sur la tige fémorale, on n'utilise que les métaux avec les inconvénients qu'ils présentent, en particulier de moins bonnes propriétés de frottement face à la cupule de PE haute densité.

On a cherché à utiliser des têtes fémorales en alumine dense pour profiter de son meilleur coefficient de frottement; mais compte tenu de ses caractéristiques mécaniques insuffisantes on ne peut pas utiliser de telles têtes fémorales avec un diamètre inférieur à 28 mm et assemblées par emmanchement conique sur la tige fémorale, sans encourir l'éclatement de ladite tête.

Ainsi, différents aménagements ou modifications dudit emmanchement conique ont été proposés pour subvenir à cette lacune. Ces aménagements consistent généralement à introduire un matériau "amortisseur de contrainte", dans l'emmanchement conique, entre la tige métallique et la tête céramique.

On peut citer par exemple :
- FR 2580170 (Flegeau) l'utilisation d'un manchon métallique intermédiaire solidarisé par différents moyens (notamment métallisation, brasure...) à une calotte en céramique, formant la tête fémorale, ledit manchon étant engagé sur la partie tronconique de la tige fémorale;
- FR 2610514 (Cuilleron) la coulée d'un métal dans une coque céramique, en ménageant dans cette masse solidifiée un évidement où est logée l'extrémité de la tige fémorale;
- FR 2391711 (Sulzer) l'utilisation d'un manchon plastique inséré entre la céramique et le métal.

Toutes ces solutions peuvent apporter des réponses au problème de la tenue mécanique des têtes de fémur en alumine dense, mais elles sont souvent difficiles à mettre en oeuvre au moment de l'opération de mise en place de la prothèse et ne satisfont pas généralement le chirurgien.

## OBJET DE L'INVENTION

C'est pourquoi la demanderesse a cherché à mettre au point une prothèse fémorale comprenant un assemblage par emmanchement conique direct entre une tête fémorale en céramique de petit diamètre d'au plus 26mm et une tige fémorale métallique, sans l'aide de pièces intermédiaires et/ou autres moyens annexes de fixation, tels que collage, brasure, tenon, etc...

Une telle prothèse n'est ainsi pas sujette à éclatement lorsqu'elle est soumise à des efforts mécaniques intenses, tout en étant simple à utiliser et mettre en place.

## DESCRIPTION DE L'INVENTION

L'invention est une prothèse fémorale constituée d'une tige métallique comportant à l'une de ses extrémités un tronc de cone mâle et d'une tête fémorale céramique comportant au moins un évidement borgne tronconique, assemblés à l'aide d'un emmanchement conique caractérisée par la combinaison de moyens suivants : ladite tête est en céramique, de préférence de zircone stabilisée dense, elle a un diamètre d'au plus 26 mm, l'angle total au sommet de l'évidement borgne tronconique servant à l'emmanchement conique est en général supérieur à 4°, le diamètre d'entrée dudit tronc de cône servant à l'emmanchement conique mesuré à la périphérie de la tête fémorale est compris entre 8 et 14 mm, de préférence entre 10 et 11 mm.

La céramique de zircone est obtenue par frittage à partir d'un mélange de poudre de zircone et d'un agent stabilisant connu par l'homme de l'art, tels que $Y_2O_3$, MgO, CaO, les oxydes de terres rares ... ou leurs mélanges, la teneur finale en $ZrO_2$ étant en général supérieure à 95% (poids). On effectue soit un frittage naturel après mise en forme et pressage à froid, soit un frittage sous charge ou mieux un frittage sous charge isostatique (HIP - Hot Isostatic Pressing) comportant éventuellement une étape de préfrittage sans charge.

On obtient ainsi une céramique de zircone stabilisée dans la phase quadratique utilisable selon l'invention. Il est préférable qu'elle présente au moins les caractéristiques physiques suivantes :

- masse spécifique supérieure à 6 $g/cm^3$, ou 98% de la densité théorique
- taille moyenne des grains (mesurée par microscopie électronique à balayage selon la méthode ASTM : NPA 04102) inférieure ou égale à 1 micromètre
- module de rupture à la flexion 3 points supérieur à 920 MPa (projet de norme française B41G Doc 12)
- module d'élasticité (méthode ASTM C 674) supérieur à 220 GPa

Une céramique frittée d'une autre nature, par exemple oxyde, carbure, nitrure, peut également être utilisée selon l'invention à condition qu'elle présente au moins les caractéristiques mécaniques de résistance à la flexion, de module d'Young, de taille de grains indiquées précédemment pour la zircone et évidemment qu'elle satisfasse aux conditions de biocompatibilité requises pour la présente application.

Avantageusement, on peut utiliser des pièces en zircone stabilisée obtenues par HIP; elles présentent, par exemple, au moins les caractéristiques suivantes :

. masse spécifique supérieure à 6 $g/cm^3$
. taille moyenne des grains inférieure à 1 micromètre
. résistance à la flexion supérieure à 1600 MPa
. module d'Young supérieur à 220 GPa.

On peut utiliser également d'autres céramiques présentant au moins les caractéristiques de l'une ou l'autre des deux séries précédemment citées. La tête fémorale est en général sphérique et a un diamètre d'au plus 26 mm; l'invention est particulièrement intéressante pour les sphères de diamètre 22,22 mm. Ladite tête comporte au moins un évidement borgne tronconique dont l'axe est de préférence radial et la demanderesse a constaté qu'il fallait de préférence utiliser un angle total au sommet d'au moins 6° pour obtenir des prothèses fémorales, avec une tête de petit diamètre (d'au plus 26mm), de solidité suffisamment élevée pour être implantée avec un risque minimum de rupture. On considère généralement que la tête fémorale doit pouvoir supporter une charge minimum d'environ 30-35 kN sans se détériorer ou se rompre. Cette charge de rupture est mesurée selon le projet de norme française PR.S 90443 du 10.2.88, où une force croissante est appliquée sur la tête fémorale assemblée à la tige métallique selon l'axe de l'assemblage.

Un accroissement notoire de solidité est obtenu de préférence en employant un angle d'environ 10°.

De même, il est essentiel que simultanément le diamètre d'entrée de cône soit tel que défini plus haut, les valeurs supérieures à 14 mm étant notamment de nature à augmenter significativement le risque de rupture de la tête fémorale.

L'évidement borgne tronconique a des parois latérales qui servent à assurer l'assemblage autobloquant par emmanchement conique en étant en contact avec les parois d'un cône mâle métallique correspondant, de préférence de même conicité; ledit cône mâle, situé à une extrémité d'une tige fémorale, dont l'autre extrémité est implantée dans le fémur, est enfoncé à force et ainsi bloqué directement dans l'évidement tronconique.

Ledit évidement borgne tronconique, servant à l'emmanchement débouche à la surface de ladite tête soit directement, soit par l'intermédiaire d'un autre tronc de cône légèrement plus ouvert, comme cela sera vu plus loin.

La tige fémorale métallique peut être en alliage de Ti, acier inox, alliage Cr-Co-Mo, ou tous autres métaux ou alliages utilisés pour réaliser des implants orthopédiques.

On peut optimiser l'assemblage de la tête fémorale sur la tige métallique par tous moyens connus et en particulier par le dispositif décrit dans la demande de brevet français 88-09042, dans lequel le contact entre le cône mâle de ladite tige et le cône femelle de ladite tête, assurant l'assemblage par emmanchement conique autobloquant, n'a lieu que dans une zone profonde tronconique de l'évidement borgne de la tête céramique.

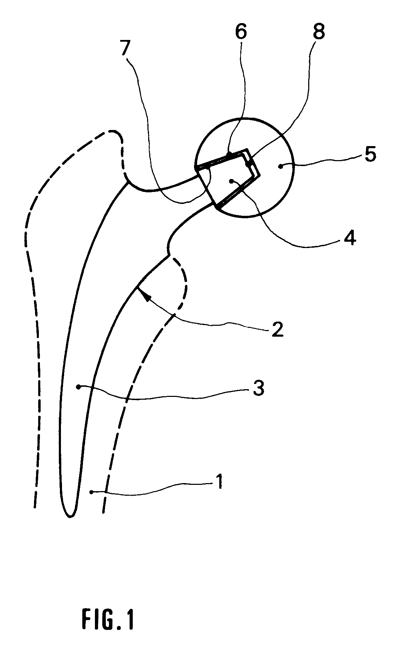

La figure 1 illustre une prothèse fémorale selon l'invention. On y voit :

- en 1 le fémur,
- en 2 la tige métallique fémorale dont l'extrémité 3 est implantée dans le fémur et dont l'extrémité 4 tronconique sert à effectuer l'assemblage par emmanchement conique avec la tête fémorale céramique 5 de petit diamètre,
- en 6 la paroi latérale de l'évidement tronconique pratiqué dans la tête fémorale 5 qui est en contact sur toute sa hauteur avec la paroi latérale 7 du cône mâle 4,
- en 8 un espace de garde entre le sommet du tronc de cône mâle 4 et le fond de l'évidement tronconique pratiqué dans la tête 5.

Dans ce cas l'évidement borgne est constitué d'un seul tronc de cône débouchant directement à la surface de la tête fémorale 5.

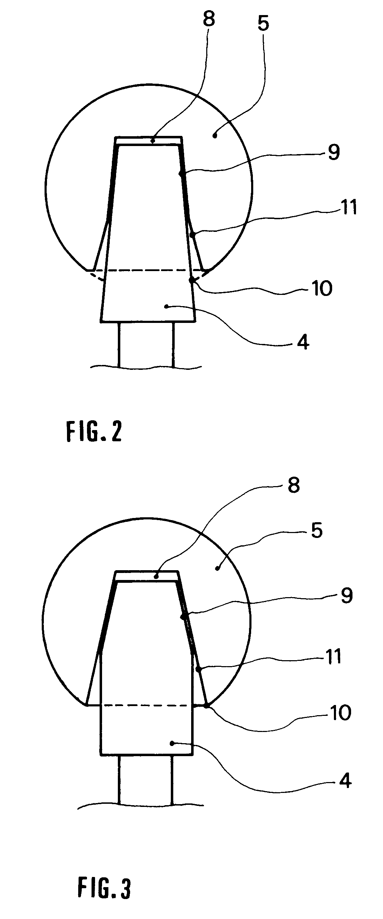

Les figures 2 et 3 illustrent des optimisations de l'emmanchement conique de l'invention effectuées selon le dispositif de la demande française n° 88-09042 d'après laquelle le contact entre les cônes mâle et femelle a lieu dans une zone "profonde" de l'évidement borgne de la tête fémorale céramique 5.

Dans la figure 2 on voit que la zone de contact par emmanchement conique autobloquant, entre la tête fémorale céramique 5 et la tige métallique fémorale 4, a lieu en 9 dans la zone "profonde" de l'évidement borgne. L'angle total au sommet selon l'invention est celui de cette zone de contact tronconique. La zone de non contact 11 est située vers l'extérieur de l'évidement borgne et est obtenue dans ce cas par un usinage plus évasé de la tête fémorale 5. Le diamètre d'entrée 10 de l'évidement borgne tronconique, où a lieu le contact, est mesuré sur le cercle obtenu par l'intersection de la surface extérieure de la tête fémorale 5 et du prolongement du tronc de cône de contact 9.

Dans la figure 3 la zone de contact 9 est toujours située dans la zone profonde de l'évidement borgne. La zone de non contact 11 est obtenue dans ce cas par un usinage en dépouille de la partie correspondante du cône mâle 4 de la tige métallique fémorale. Le diamètre d'entrée 10 de l'évidement borgne tronconique de contact est également mesuré sur le cercle intersection dudit évidement tronconique avec la surface extérieure de la tête fémorale.

## EXEMPLES

Pour illustrer l'intérêt de l'invention des essais de rupture de la tête fémorale céramique de différentes prothèses ont été réalisés selon le projet de norme PRS 90443 "Pièces fémorales de prothèse de hanche avec tête rapportée : spécification de la tête et de la partie mâle de l'emboîtement". Le principe de l'essai consiste à appliquer une charge sur la tête céramique selon l'axe de l'assemblage jusqu'à obtenir la ruine de ladite tête.

### Exemple 1

On a réalisé des prothèses à l'aide de tête fémorale en alumine dense; les caractéristiques mécaniques de cette alumine sont conformes à la norme ASTM F603 ou ISO 6474.

Pour illustrer les problèmes posés par l'adaptation de ce matériau à l'obtention de certains types de prothèse fémorale, on a monté des têtes fémorales en alumine de petit diamètre (26 mm) sur une tige fémorale métallique à l'aide d'un emmanchement cônique du type de celui de la figure 1 (contact entre cône mâle et évidement borgne sur la totalité des parois dudit évidement borgne de la tête fémorale) en utilisant le même angle pour l'évidement borgne et le cône mâle.

Les caractéristiques géométriques sont les suivantes :

. diamètre de la tête fémorale : 26mm

. angle total au sommet de l'évidement : 6°

. diamètre d'entrée du cône : 14mm

Les essais effectués ont donné une rupture pour 25 kN, valeur jugée insuffisante pour effectuer une implantation sans risque.

### Exemple 2

Dans cet exemple destiné à illustrer l'invention, les têtes fémorales sont en zircone frittée dense obtenue par frittage naturel, dont la qualité est conforme aux caractéristiques données plus haut dans la description. Les têtes fémorales ont un diamètre de 26mm et l'emmanchement conique est identique à celui de l'Exemple 1. Les résultats des essais sont donnés dans le tableau suivant :

| Essais (26mm) | Angle au sommet (°) | Diamètre d'entrée de cône (mm) | Charge à la rupture (kN) |
|---|---|---|---|
| 1 | 6 | 14 | 43 |
| 2 | 6 | 12 | 50 |
| 3 | 10 | 11 | 61 |

On peut voir que selon l'invention, on note dans l'essai 1 une charge de rupture améliorée qui rend la prothèse implantable, la résistance à la rupture dépassant assez nettement le seuil limite de 30-35 kN.

On voit également un résultat particulièrement amélioré avec l'essai 3 (angle de 10° et diamètre d'entrée de cône de 11 mm).

Exemple 3

Cet exemple illustre également l'invention. Dans ces essais la tête fémorale a un diamètre de 22,22mm et l'emmanchement conique est du type de celui décrit dans la figure 2 : contact du cône mâle sur une partie seulement des parois de l'évidement borgne, par évasement de la partie antérieure dudit évidement.

Deux types de céramiques ont été utilisés : zircone dense identique à celle de l'Exemple 2 obtenue par frittage naturel, zircone obtenue par H.I.P. ayant les caractéristiques correspondantes données dans la description.

Les résultats obtenus sont les suivants :

| Essais (22,22mm) | Angle au sommet (°) | Diamètre d'entrée de cône (mm) | Charge à la rupture (kN) |
|---|---|---|---|
| 4 (zircone frittage naturel) | 10 | 10 | 82 |
| 5 (zircone HIP) | 10 | 10 | 120 |

Ces essais montrent que même avec des têtes fémorales de petit diamètre on obtient selon l'invention des charges de rupture très élevées.

Ainsi, les caractéristiques combinées de l'invention permettent d'utiliser des têtes fémorales de petit diamètre (d'au plus 26mm et notamment 22,22mm) pour réaliser des prothèses fémorales, par emmanchement conique sur une tige métallique, qui ne présentent pas de risques de rupture après implantation, alors qu'un tel résultat n'est pas possible avec des têtes fémorales de l'art antérieur en alumine dense.

**Revendications**

1. Prothèse fémorale constituée d'une tige métallique comportant à l'une de ses extrémités un tronc de cône mâle, et d'une tête fémorale céramique comportant au moins un évidement borgne tronconique, assemblés à l'aide d'un emmanchement conique caractérisée par la combinaison de moyens suivants : ladite tête est en céramique, elle a un diamètre d'au plus (maxi) 26 mm, l'angle total au sommet de l'évidement borgne tronconique servant à l'emmanchement conique est supérieur à 4°, le diamètre d'entrée dudit tronc de cône servant à l'emmanchement conique mesuré à la périphérie de la tête fémorale est compris entre 8 et 14 mm, de préférence entre 10 et 11 mm.

2. Prothèse selon la revendication 1, caractérisée en ce que la tête fémorale est en céramique de zircone stabilisée dense.

3. Prothèse selon la revendication 2, caractérisée en ce que la céramique de zircone stabilisée contient plus de 95% (poids) de $ZrO_2$.

4. Prothèse selon l'une quelconque des revendications 1 à 3 caractérisée en ce que l'angle total au sommet est supérieur à 6° et de préférence égal à environ 10°.

5. Prothèse selon l'une quelconque des revendications 1 à 4 caractérisée en ce que le diamètre d'entrée de cône est de 10 mm.

6. Prothèse selon l'une quelconque des revendications 1 à 5 caractérisée en ce que le diamètre de la tête céramique est de 22,22 mm.

7. Prothèse selon l'une quelconque des revendications 1 à 6 caractérisée en ce que la céramique de la tête fémorale présente au moins les caractéristiques mécaniques suivantes :
   . masse spécifique supérieure à 6 $g/cm^3$
   . taille moyenne des grains inférieureou égale à 1 micromètre
   . résistance à la flexion supérieure à 920 MPa
   . module d'élasticité supérieur à 220 GPa.

8. Prothèse selon la revendication 7 caractérisée en ce que la céramique de la tête fémorale présente au moins les caractéristiques mécaniques suivantes :
   . masse spécifique supérieure à 6 $g/cm^3$
   . taille moyenne des grains inférieure à 1 micromètre
   . résistance à la flexion supérieure à 1600 MPa
   . module d'Young supérieur à 220 GPa.

9. Prothèse selon l'une quelconque des revendications 1, 4 à 8 caractérisée en ce que la tête fémorale est en céramique d'oxyde, carbure, nitrure ou borure, ayant au moins les caractéristiques mécaniques de l'une quelconque des revendications 7 ou 8.

10. Prothèse selon l'une quelconque des revendications 1 à 9 caractérisée en ce que la tête fémorale céramique est obtenue par HIP (Hot Isostatic Pressing).

11. Prothèse selon l'une quelconque des revendications 1 à 10 caractérisée en ce que l'emmanchement conique se fait par contact entre le cône mâle de la tige fémorale et une partie seulement de l'évidement borgne située dans une zone profonde dudit évidement, la partie antérieure dudit évidement étant une zone de non contact.


## Claims

1. A femoral prosthesis consisting of a metal rod comprising at one of its ends a male truncated cone and a ceramic femoral head comprising at least one frustoconical blind recess, assembled by means of a conical sleeve-like fitting, characterised by the combination of the following means: the said head is of ceramic material, it has a maximum diameter of 26 mm, the total angle at the apex of the frustoconical blind recess serving for the conical fitment is greater than 4°, the diameter of the entrance of the said truncated cone serving for conical sleeve-like fitment, measured at the periphery of the femoral head, is comprised between 8 and 14 mm and is preferably between 10 and 11 mm.

2. A prothesis according to claim 1, charaterised in that the femoral head is of stabilised dense zirconia ceramic material.

3. A prosthesis according to claim 2, characterised in that the stabilised zirconia ceramic material contains more than 95% (by weight) $ZrO_2$.

4. A prosthesis according to any one of claims 1 to 3, characterised in that the total angle at the apex is greater than 6° and is preferably equal to about 10°.

5. A prosthesis according to any one of claims 1 to 4, characterised in that the diameter at the entrance to the cone is 10 mm.

6. A prosthesis according to any one of claims 1 to 5, characterised in that the diameter of the ceramic head is 22.22 mm.

7. A prosthesis according to any one of claims 1 to 6, characterised in that the ceramic material of the femoral head has at least the following mechanical properties:
   . specific mass greater than 6 g/cu.cm
   . medium size of grains less than or equal to 1 micron
   . resistance to flexion greater than 920 MPa
   . elasticity modulus greater than 220 GPa.

8. A prosthesis according to claim 7, characterised in that the ceramic material of the femoral head has a least the following mechanical properties:
   . specific mass greater than 6 g/cu.cm
   . medium size of grains less than 1 micron
   . resistance to flexion greater than 1600 MPa
   . Young's modulus greater than 220 GPa.

9. A prosthesis according to any one of claims 1, 4 to 8, characterised in that the femoral head is of oxide, carbide, nitride or boride ceramic material having at least the mechanical properties of any one of claims 7 or 8.

10. A prosthesis according to any one of claims 1 to 9, characterised in that the ceramic femoral head is obtained by HIP (Hot Isostatic Pressing).

11. A prosthesis according to any one of claims 1 to 10, characterised in that the conical sleeve-like fitting is carried out by contact between the male cone of the femoral rod and only a part of the blind access situated in a deep zone in the said recess, the front part of the said recess being a non-contact zone.


**Patentansprüche**

1. Femoralprothese, bestehend aus einem Metallschaft, der an einem seiner Enden einen positiven Kegelstumpf trägt, und aus einem Femurkopf aus Keramikmaterial, der mindestens eine blinde kegelstumpfartige Aussparung aufweist, die mittels eines konischen Einsteckendes zusammengebaut sind, gekennzeichnet durch die Kombination folgender Merkmale: der Kopf besteht aus Keramikmaterial, hat einen Durchmesser von höchstens (maximal) 26 mm, der Gesamtwinkel am Scheitel der blinden kegelstumpfartigen Aussparung, die dem konischen Einsteckende dient, beträgt mehr als 4°, und der am Umfang des Femurkopftes gemessene Eintrittsdurchmesser des als konisches Einsteckende dienenden Kegelstumpfes beträgt zwischen 8 und 14 mm, vorzugsweise zwischen 10 und 11 mm.

2. Prothese nach Anspruch 1, dadurch gekennzeichnet, daß der Femurkopf aus stabilisiertem dichtem Zirkoniumkeramikmaterial besteht.

3. Prothese nach Anspruch 2, dadurch gekennzeichnet, daß das stabilisierte Zirkoniumkeramikmaterial mehr als 95 Gew.-% $ZrO_2$ enthält.

4. Prothese nach irgendeinem der Ansprüche 1 bis 3, dadurch gekennzeichnet, daß der Gesamtwinkel am Scheitel mehr als 6° beträgt und vorzugsweise etwa gleich 10° ist.

5. Prothese nach irgendeinem der Ansprüch 1 bis 4, dadurch gekennzeichnet, daß der Eintrittsdurchmesser des Konus 10 mm beträgt.

6. Prothese nach inrgendeinem der Ansprüche 1 bis 5, dadurch gekennzeichnet, daß der Durchmesser der Keramikkopfes 22,22 mm beträgt.

7. Prothese nach irgendeinem der Ansprüche 1 bis 6, dadurch gekennzeichnet, daß das Keramikmaterial des Femurkopfes mindestens die folgenden mechanischen Eigenschaften aufweist:
   - spezifische Masse: größer als 6 g/cm³,
   - mittlere Korngröße: kleiner als oder gleich 1 Mikrometer,
   - Biegefestigkeit: größer als 920 MPa,
   - Elastizitätsmodul: größer als 220 GPa.

8. Prothese nach Anspruch 7, dadurch gekennzeichnet, daß das Keramikmaterial des Femurkopfes mindestens die folgenden mechanischen Eigenschaften aufweist:
   - spezifische Masse: größer als 6 g/cm³
   - mittlere Korngröße: kleiner als 1 Mikrometer,
   - Biegefestigkeit: größer als 1600 MPa,
   - Young-Modul: größer als 220 GPa.

9. Prothese nach irgendeinem der Ansprüche 1 und 4 bis 8, dadurch gekennzeichnet, daß der Femurkopf aus Oxid-, Carbid-, Nitrid- oder Boridkeramikmaterial besteht und mindestens die in einem der Ansprüche 7 oder 8 gennanten mechanischen Eigenschaften aufweist.

10. Prothese nach irgendeinem der Ansprüche 1 bis 9, dadurch gekennzeichnet, daß der Femurkopf aus dem Keramikmaterial durch HIP-Methode (Hot Isostatic Pressing) erhalten worden ist.

11. Prothese nach irgendeinem der Ansprüche 1 bis 10, dadurch gekennzeichnet, daß das konische Einsteckende den Kontakt zwischen dem positiven Konus des Femurschaftes und nur einem Teilstück der blinden Aussparung, das in einer tiefen Zone dieser Aussparung angeordnet ist, herbeiführt, wobei das vordere Teilstück dieser Aussparung eine Zone ohne Kontakt ist.

FIG.1

FIG. 2

FIG. 3